# EUROPEAN PATENT APPLICATION

(11) **EP 3 146 931 A1**
(43) Date of publication of application: **29.03.2017**
(21) Application number: 14859318.9
(22) Date of filing: 22.09.2014
(51) Int. Cl.: A61B 90/00

(54) **HEALTH EXAMINATION CONTROL DEVICE, HEALTH TELEVISION SYSTEM AND HEALTH EXAMINATION METHOD**

(30) Priority: 23.05.2014 CN 201410220534
(71) Applicant: BOE Technology Group Co., Ltd., Beijing 100015 (CN)
(72) Inventor: ZHAO, Tianyue, Beijing 100176 (CN)
(74) Representative: Klunker . Schmitt-Nilson . Hirsch
(86) International application number: PCT/CN2014/087021
(87) International publication number: WO 2015/176434

(57) **Abstract**

A health examination control apparatus, a health television system and a health examination method are provided. The health examination control apparatus comprises a request processing device (101), a recognition device (102) and a health examination device (103); the request processing device (101) is configured to receive incoming information and send a health examination request instruction to the recognition device (102); the recognition device (102) is configured to receive the health examination request instruction and send a health examination start instruction to the health examination device (103); and the health examination device (103) is configured to receive health examination information and output corresponding health examination result information after receiving the health examination health start instruction. The health examination control apparatus starts a health examination by means of external incoming remind, avoids a case that a user does not do health examination in time due to forgetfulness or lack of attention to own physical status, is simple to operate and has high practicability.

## Description

### TECHNICAL FIELD

At least one embodiment of the present disclosure relates to a health examination control apparatus, a health television system and a health examination method.

### BACKGROUND

Health television has been gradually widespread. But as time goes on, a lot of functions tend to be gradually ignored by people, many people are concerned about their own bodies only in the case of illness, and the senior elders are forgetful, so effective health examinations often cannot be performed in time.

### SUMMARY

At least one embodiment of the present disclosure provides a health examination control apparatus, a health television system and a health examination method to avoid the case that users do not do health examination in time due to forgetfulness or lack of attention to their own physical statuses.

At least one embodiment of the present disclosure provides a health examination control apparatus, which comprises a request processing device, a recognition device and a health examination device. The request processing device is configured to receive incoming information and send a health examination request instruction to the recognition device; the recognition device is configured to receive the health examination request instruction and send a health examination start instruction; and the health examination device is configured to receive health examination information and output corresponding health examination result information after receiving the health examination start instruction.

At least one embodiment of the present disclosure further provides a health television system, which comprises the above-mentioned health examination control apparatus.

At least one embodiment of the present disclosure further provides a health examination method. The method comprises: receiving incoming information and sending a health examination request instruction; sending a health examination start instruction after receiving the health examination request instruction; and receiving health examination information and outputting corresponding health examination result information after receiving the health examination start instruction.

### BRIEF DESCRIPTION OF THE DRAWINGS

In order to clearly illustrate the technical solution of the embodiments of the disclosure, the drawings of the embodiments will be briefly described in the following; it is obvious that the described drawings are only related to some embodiments of the disclosure and thus are not limitative of the disclosure.
FIG. 1 is a basic schematic structural view of a health examination control apparatus provided by an embodiment of the present disclosure;
FIG. 2 is a schematic structural view of a health examination control apparatus provided by an embodiment of the present disclosure; and
FIG. 3 is a basic flow chart of a health examination method provided by an embodiment of the present disclosure.

### DETAILED DESCRIPTION

In order to make objects, technical details and advantages of the embodiments of the disclosure apparent, the technical solutions of the embodiments will be described in a clearly and fully understandable way in connection with the drawings related to the embodiments of the disclosure. Apparently, the described embodiments are just a part but not all of the embodiments of the disclosure. Based on the described embodiments herein, those skilled in the art can obtain other embodiment(s), without any inventive work, which should be within the scope of the disclosure.

The inventor of the application noted that many health examination devices are complex and passive in operation, and this makes people unable to know their own physical statuses at any time and unable to timely remind the family of health examination.

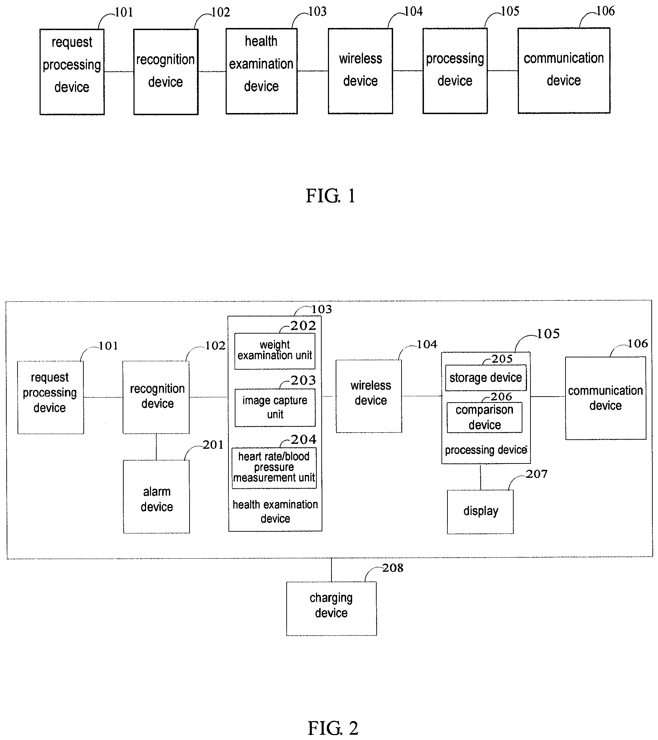

At least one embodiment of the present disclosure firstly provides a health examination control apparatus at first. As illustrated in FIG. 1, the health examination control apparatus comprises: a request processing device 101 configured to receive incoming information and send a health examination request instruction to a recognition device 102; the recognition device 102 configured to receive the health examination request instruction and send a health examination start instruction to a health examination device 103; and the health examination device 103 configured to receive health examination information and output corresponding health examination result information after receiving the health examination start instruction. In the embodiment of the present disclosure, the recognition device 102 sends the health examination start instruction to the health examination device 103 which hence starts a health examination operation, receives health information (namely health examination information) obtained from the health examination operation, and outputs corresponding health examination result.

In one embodiment, the health examination control apparatus can further comprise: a wireless device 104 configured to transmit the health examination result information.

In one embodiment, the health examination control apparatus can further comprise: a processing device 105 configured to generate information of a health examination report according to the health examination result information.

In one embodiment, the health examination control apparatus can further comprise: a communication device 106 configured to communicate with a communication network and configured to send the information of the health examination report to the communication network.

It should be noted that the health examination control apparatus provided by embodiments of the present disclosure can comprise at least one selected from the group consisting of the wireless device, the processing device and the communication device. The incoming information can be received through a subscriber identity module (SIM) card to achieve a communication function. But embodiments of the present disclosure are not limited thereto. For instance, the incoming information can also be received through internet. It can be seen that, in the health examination control apparatus provided by embodiments of the present disclosure, the apparatus is started by external incoming remind to start a health examination, so that a passive health examination can be converted into an active health examination, and hence the case that users do not do health examination in time due to forgetfulness or lack of attention to their own physical statuses can be avoided. Moreover, the health examination control apparatus is simple to operate and has high practicability.

In one embodiment, the health examination control apparatus provided by embodiments of the present disclosure can start a health examination through an incoming remind from a friend or a relative of the user and can give an alarm when the recognition device 102 fails to start the health examination device 103 properly to do health examination. By adoption of the setting, the user can be well supervised. For instance, the health examination control apparatus can further comprise: an alarm device 201 which, as illustrated in FIG. 2, is connected with the recognition device 102 and configured to send an alarm information when the health examination start instruction is not received within a certain period of time. For instance, the alarm device 201 can include an indicator light and/or a buzzer.

The health examination device 103 can examine a plurality of health information of a human body. For instance, the health examination device 103 can further include: a weight examination unit 202, and/or an image capture unit 203, and/or a heart rate/blood pressure measurement unit 204, as illustrated in FIG. 2. Correspondingly, in one embodiment, the health examination result information can further include: weight result information, and/or body height result information, and/or heart rate and/or blood pressure result information of a human body.

For instance, the weight examination unit 202 can include a pressure sensor and is configured to obtain weight information according to the pressure that the human body applies to the pressure sensor and output the weight result information.

For instance, the image capture unit 203 can be configured to obtain body height information by calculation according to positioned body image information and output the body height result information.

For instance, the heart rate/blood pressure measurement unit 204 can include a wrist sensor and is configured to obtain heart rate and/or blood pressure information of the human body according to detection information that the wrist sensor obtains from the wrist of the human body and output the heart rate and/or blood pressure result information of the human body.

In one embodiment, the wireless device 104 can transmit information through a plurality of wireless communication means. For instance, the wireless device 104 can comprise: an infrared device, a bluetooth device, a WIFI (Wireless Fidelity) device or a GPS (Global Positioning System) device.

In one embodiment, the processing device 105 can process the acquired health examination result information. For instance, the processing device 105 can further include: a storage device 205 configured to store standard health information; and a comparison device 206 configured to compare the received health examination result information with the standard health information and generate the information of the health examination report according to information obtained after comparison.

For instance, a user can acquire the process and the result of a health examination through a display device. For instance, the health examination control apparatus can further comprise: a display 207 configured to display the health examination report according to the received information of the health examination report. For instance, the display 207 can be connected with the processing device 105. For instance, the display 207 can also be directly connected with the health examination device 103. In this way, the health examination result information outputted by the health examination device 103 can be displayed by the display 207 directly.

In one embodiment of the present disclosure, a base for the placement of the health examination control apparatus can be adopted for charging. For instance, the health examination control apparatus can further comprise: a charging device 208, including the base for the placement of the apparatus and being configured to charge the apparatus through a contact on the base. Therefore, the battery replacement operation can be avoided, and hence the space can be saved.

The above-mentioned health examination control apparatus provided by embodiments of the present disclosure can be a remote controller, a telephone set or the like or other controller. The health examination control apparatus can be integrally formed and can also be formed by a plurality of separate parts as long as the above functional units are provided. The above functional units can be achieved by those skilled in the art by adoption of hardware, software or firmware or a combination thereof, and hence the health examination control apparatus provided by embodiments of the present disclosure can be formed.

At least one embodiment of the present disclosure further provides a health television system, which comprises the health examination control apparatus in any one of the above-mentioned embodiments.

In the health examination system provided by the embodiment of the present disclosure, when video is played in the process of starting a health examination, the video can be interruptted and switched in a forced way to start the health examination; and when the health television system is in an off state, the health television system can be started in a forced way to perform the health examination. For instance, the recognition device 102 can also be configured to: send shielding information for shielding a video that the health television system plays at the same time when the health examination start instruction is sent; and/or send start information for the forced startup of the health television system.

After the health examination process is over, the health television system can be restored to the initial state by sending recovery information: when a video is played before the health examination, the video play is restored after the health examination process is over; and when the health television system is in the off state before the health examination, the health television system is switched off after the health examination process is over. For instance, the health television system can further comprise: a recovery device configured to send recovery information after information of a health examination report is sent to the communication network.

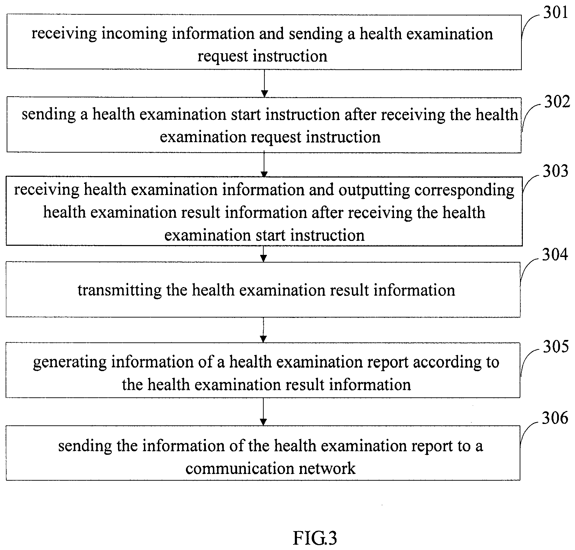

At least one embodiment of the present disclosure further provides a health examination method. As illustrated in FIG. 3, the method comprises the following steps:
S301: receiving incoming information and sending a health examination request instruction;
S302: sending a health examination start instruction after receiving the health examination request instruction; and
S303: receiving health examination information and outputting corresponding health examination result information after receiving the health examination start instruction.
   In various embodiments, the health examination method can further comprise at least one selected from the group consisting of steps 304 to 306.
S304: transmitting the health examination result information.
S305: generating information of a health examination report according to the health examination result information.
S306: sending the information of the health examination report to a communication network.

In the method provided by embodiments of the present disclosure, a plurality of health examination information can be acquired. For instance, the step of receiving the health examination information and outputting corresponding health examination result information can include: obtaining weight information according to the pressure that the human body applies to a pressure sensor and outputting weight result information; and/or obtaining body height information according to positioned body image information and outputting body height result information; and/or obtaining heart rate and/or blood pressure information of the human body according to detection information of a wrist sensor and outputting heart rate and/or blood pressure result information of the human body.

In a method provided by one embodiment, the health examination result can also be analyzed to acquire the health condition of the human body. For instance, the fat content of the human body is determined through weight and height, and hence corresponding health condition and exercising way are determined; and the variation in the physical health status is monitored by combination of data such as heart rate and blood pressure for early prevention and timely treatment.

In the health examination control apparatus, the health television system and the health examination method, provided by embodiments of the present disclosure, the apparatus is started through external incoming remind to start a health examination, so that a passive health examination can be converted into an active health examination, and hence the case that users do not do health examination in time due to forgetfulness or lack of attention to their own physical statuses can be avoided. Moreover, embodiments of the present disclosure are simple to operate and have high practicability.

It should be finally noted that: the above embodiments are only used to illustrate the technical solutions of the disclosure rather than limitations; although the disclosure has been illustrated in detail with reference to the above embodiments, those skilled in the art should understand that the technical solutions recited in each of the above embodiments can be still modified, or part of the technical features may be varied equivalently; but these modifications or variations do not make the substance of the corresponding technical solutions go beyond of the spirit and scope of the technical solution of each embodiment of the disclosure.

The application claims priority to the Chinese Patent Application No. 201410220534.5, filed May 23, 2014, which is hereby entirely incorporated by reference as a part of the present application.

## Claims

1. A health examination control apparatus, comprising: a request processing device, a recognition device and a health examination device, wherein
the request processing device is configured to receive incoming information and send a health examination request instruction to the recognition device;
the recognition device is configured to receive the health examination request instruction and send a health examination start instruction to the health examination device; and
the health examination device is configured to receive health examination information and output corresponding health examination result information, after receiving the health examination start instruction.

2. The health examination control apparatus according to claim 1, further comprising: a wireless device configured to transmit the health examination result information.

3. The health examination control apparatus according to claim 2, further comprising: a processing device configured to generate information of a health examination report according to the health examination result information.

4. The health examination control apparatus according to claim 3, further comprising: a communication device configured to communicate with a communication network and configured to send the information of the health examination report to the communication network.

5. The health examination control apparatus according to any one of claims 1 to 4, further comprising: an alarm device connected with the recognition device and configured to send alarm information where the health examination start instruction is not received within a certain period of time.

6. The health examination control apparatus according to claim 5, wherein the alarm device comprises an indicator light and/or a buzzer.

7. The health examination control apparatus according to any one of claims 1 to 6, wherein the health examination device comprises: a weight examination unit, and/or an image capture unit, and/or a heart rate/blood pressure measurement unit; the health examination result information comprises: weight result information, and/or body height result information, and/or heart rate and/or blood pressure result information of a human body;
the weight examination unit comprises a pressure sensor and is configured to obtain weight information according to pressure that the human body applies to the pressure sensor and output the weight result information;
the image capture unit is configured to obtain body height information according to positioned body image information and output the body weight result information; and
the heart rate/blood pressure measurement unit comprises a wrist sensor and is configured to obtain heart rate and/or blood pressure information of the human body according to detection information of the wrist sensor and output the heart rate and/or blood pressure result information of the human body.

8. The health examination control apparatus according to any one of claims 2 to 4, wherein the wireless device comprises an infrared device, a bluetooth device, a WIFI device or a GPS device.

9. The health examination control apparatus according to claim 3 or 4, wherein the processing device comprises:
a storage device configured to store standard health information; and
a comparison device configured to compare the received health examination result information with the standard health information and generate the information of the health examination report according to information obtained after comparison.

10. The health examination control apparatus according to any one of claims 3, 4 and 9, further comprising:
a display connected with the processing device and configured to display the health examination report according to the information of the health examination report received.

11. The health examination control apparatus according to any one of claims 1 to 10, further comprising:
a charging device, comprising a base configured for placement of the health examination control apparatus and being configured to charge the health examination control apparatus through a contact on the base.

12. A health television system, comprising a health examination control apparatus according to any one of claims 1 to 11.

13. The health television system according to claim 12, wherein the recognition device is further configured to:
send shielding information for shielding a video, which is played by the health examination system upon the health examination start instruction being sent; and/or
send start information for forced startup of the health television system.

14. The health television system according to claim 12 or 13, wherein the health television system further comprises: a recovery device configured to send recovery information after information of a health examination report is sent to a communication network.

15. A health examination method, comprising:
receiving incoming information and sending a health examination request instruction;
sending a health examination start instruction after receiving the health examination request instruction; and
receiving health examination information and outputting corresponding health examination result information after receiving the health examination start instruction.

16. The health examination method according to claim 15, further comprising: transmitting the health examination result information.

17. The health examination method according to claim 16, further comprising: generating information of a health examination report according to the health examination result information.

18. The health examination method according to claim 17, further comprising: sending the information of the health examination report to a communication network.

19. The health examination method according to any one of claims 15 to 18, wherein receiving the health examination information and outputting the corresponding health examination result information further comprises:
obtaining weight information according to pressure that a human body applies to a pressure sensor and outputting weight result information; and/or
obtaining body height information according to positioned body image information and outputting body height result information; and/or
obtaining heart rate and/or blood pressure information of the human body through detection information of a wrist sensor and outputting heart rate and/or blood pressure result information of the human body.
